# EUROPÄISCHE PATENTANMELDUNG

(11) **EP 2 712 929 A1**
(43) Veröffentlichungstag der Anmeldung: **02.04.2014**
(21) Anmeldenummer: 13186221.1
(22) Anmeldetag: 26.09.2013
(51) Int. Cl.: C12Q 1/04, C12Q 1/10

(54) **Verfahren zum Erzeugen eines Probensatzes für ein Nachweisverfahren für Salmonella enterica ssp. enterica**

(30) Priorität: 27.09.2012 DE 102012217646
(71) Anmelder: BCD Baltic Customized Diagnostics GmbH, 24118 Kiel (DE)
(72) Erfinder: Seegert, Dirk, 24229 Dänischenhagen (DE)
(74) Vertreter: Eisenführ Speiser

(57) **Zusammenfassung**

Verfahren zum Erzeugen eines Probensatzes für ein Nachweisverfahren für Sal*monella enterica* ssp. *enterica,* umfassend die Schritte:
a) Gewinnen einer Probe, die potentiell *Salmonella enterica* enthält,
b) Inkubieren der Probe in einem Voranreicherungsmedium für *Salmonella enterica,* so dass eine Anreicherung an Mikroorganismen erfolgt,
c) Inkubieren einer Probe des Voranreicherungsmediums in wenigstens einem Anreicherungsmedium für *Salmonella enterica,* so dass eine Anreicherung von *Salmonella enterica* erfolgt sofern vorhanden und
d) Gewinnen je einer Probe aus dem Voranreicherungsmedium und dem Anreicherungsmedium oder den Anreicherungsmedien zum Einsatz in einem spezifischen Nachweisverfahren.

## Beschreibung

Die Erfindung betrifft ein Verfahren zum Erzeugen eines Probensatzes für ein Nachweisverfahren für *Salmonella enterica* ssp. *enterica,* ein Nachweisverfahren für die entsprechenden Keime, sowie die Verwendung eines mit dem erfindungsgemäßen Verfahren erzeugten Probensatzes für ein entsprechendes Nachweisverfahren. Die Erfindung betrifft ferner ein Kit umfassend Nährmedien, die speziell für das erfindungsgemäße Verfahren zum Erzeugen eines Probensatzes eingesetzt werden können.

Salmonellen sind eine Gattung von Mikroorganismen, die Krankheiten bei Menschen verursachen. Während ein Teil der Salmonellen ausschließlich Menschen befällt (z.B. *Salmonella typhi*) haben eine Vielzahl dieser Mikroorganismen auch Tiere als Wirt und können sowohl den Menschen als auch das Tier befallen. Salmonellen kommen u.a. in Geflügelprodukten, z.B. in Frischfleich oder Eiern, vor. Die Infektion von Menschen mit Salmonellen über Lebensmittel tierischen Ursprungs ist aktuell der häufigste Infektionsweg.

Durch Salmonellen ausgelöste Krankheiten beim Menschen (Salmonellosen) gehören in Deutschland zu den meldepflichtigen Erkrankungen. Salmonellen sind außerhalb des menschlichen bzw. tierischen Körpers über einen langen Zeitraum lebensfähig. So sind sie in getrocknetem Kot zum Beispiel über 2,5 Jahre lang nachweisbar. Durch Einfrieren werden die Bakterien nicht abgetötet.

Vor diesem Hintergrund ist in vielen Bereichen, insbesondere in Bereichen von Lebens-und Futtermitteln, ein regelmäßiges Testen auf das Vorhandensein von Salmonellen vorgeschrieben. Dabei ist es schon aus wirtschaftlichen Gesichtspunkten wichtig, dass die angewendeten Testverfahren schnell und zuverlässig durchgeführt werden können. Diese beiden Gesichtspunkte sind Gesichtspunkte, die sich tendenziell widersprechen. Dementsprechend besteht ein ständiger Bedarf an effektiven, schnellen und vor allen Dingen zuverlässigen Testmethoden für den Nachweis des Vorhandenseins (oder dem Ausschluss des Vorhandenseins) von Salmonellen, insbesondere solchen, die von Tieren bzw. Tierprodukten auf den Menschen übertragen werden können.

Nach der derzeitigen Nomenklatur gibt es für die Gattung *Salmonella* drei Spezies, nämlich *S. enterica, S. bongori und S. subterranea.* Hierbei wird besonders auf *Salmonella enterica* getestet, wobei diese Spezies in sechs Sub-Spezies unterteilt wird, von denen die Sub-Spezies *enterica* die meisten für den Menschen relevanten Keime umfasst. Innerhalb der Sub-Spezies werden die Salmonellen nach dem sogenannten Kauffmann-White-Schema in insgesamt mehr als 2.500 Salmonella Serovare unterteilt, die sich aufgrund des Vorkommens von unterschiedlichen O- und H-Antigenen unterscheiden. Die Kurzschreibweise für die hier relevanten Salmonella Serovare ist zum Beispiel *S. typhimurium* für *Salmonella enterica* ssp. *enterica* Serovar typhimurium.

Für die Gesundheit des Menschen besonders bedeutsame Serovare sind *Salmonella enteritidis* und *Salmonella typhimurium,* die beim Menschen meist spontan ausheilende Durchfallerkrankungen verursachen. Allerdings können Salmonellen bei Risikogruppen wie Säuglingen, Kleinkindern, alten Menschen und immungeschwächten Patienten schwere Erkrankungen hervorrufen.

Nach dem aktuellen Stand von Wissenschaft und Forschung wird der Nachweis von Salmonellen aus Lebens- und Futtermitteln, Human- und Veterinärdiagnostikmaterial sowie aus Hygieneumfeld- und Wasserproben mit einer nicht-selektiven Voranreicherung in gepuffertem Peptonwasser (buffered peptone water, BPW) begonnen. Es gibt praktisch keine Ausnahmen davon bis auf einige auf dem Markt erhältliche kommerzielle, semi-selektive Medien, die die Eigenschaften von gepuffertem Peptonwasser und nachfolgender Selektivanreicherung vereinen sollen. Diese Spezialmedien sind teuer, die Rezeptur geschützt oder nicht veröffentlicht und finden daher nur sehr geringe Verbreitung. Das sehr nährstoffreiche BPW bewirkt in erster Linie, dass z.B. subletal geschädigte Salmonellen (wie auch andere Bakterien-Spezies) in eine aktive Wachstumsphase kommen, die den anschließenden spezifischen kulturellen Nachweis ermöglicht. Weitere nicht-selektive Anreicherungsmedien werden zwar im Zusammenhang mit Salmonellen als Enterobakterien angeboten, finden aber selten für den gezielten Nachweis von Salmonellen Verwendung.

Die in der EU aktuell maßgebende DIN EN ISO 6579:2002+A1:2007 ("Mikrobiologie von Lebensmitteln und Futtermitteln - Horizontales Verfahren zum Nachweis von Salmonella spp.") sieht nach der Voranreicherung in BPW eine Zweitanreicherung in zwei verschiedenen selektiven Flüssigmedien vor. Es wird sowohl in Rappaport-Vassiliadis Medium mit Soja (RVS) als auch in Müller-Kauffmann Tetrathionat-Novobiocin-Bouillon (MKTTn) passagiert und einen weiteren Tag inkubiert, bevor der eigentliche Speziesnachweis sowohl durch Ausstrich je auf Xylose-Lysin-Desoxycholate-Agar (XLD) als auch auf Rambach-Medium beginnt. Nach einem weiteren Tag Inkubation erfolgt die endgültige Spezies- und Serovar-Differenzierung durch biochemische Tests nach Subkultivierung von einzelnen Kolonien, wobei als Proben stets nur Fraktionen des jeweils letzten Kulturschrittes verwendet werden.

Die üblichen Abwandlungen dieser Nachweismethode (offizielle Methoden anderer nicht-EU-Staaten, "Hausmethoden") erhöhen die Sensitivität insbesondere zum Nachweis von erheblich sublethal geschädigten Salmonellen oder in schwierigen - das bakterielle Wachstum hemmende - Probenmatrices. Abwandlungen liegen v.a. in Form von bis zu fünffach aufeinander folgende Subkultivierungen über je einen ganzen Tag in z.B. einfachem Rappaport-Vassiliadis Medium (RV) oder anderen selektiven Alternativmedien vor.

Problematisch bei den derzeit durchgeführten Anreicherungsregimen für die Nachweismethode ist, dass die Anreicherung verhältnismäßig lange dauert und die Ergebnisse hinsichtlich ihrer Aussagesicherheit noch steigerbar sind. Dabei sind falsch positive Testergebnisse aus wirtschaftlicher Sicht kritisch, da beispielsweise bei Nachweis von Salmonellen betroffene Lebensmittel nicht in den Handel gelangen dürfen. Kritischer sind aber selbstverständlich falsch negative Bestimmungen, da in solchen Fällen die Gesundheit von Verbrauchern gefährdet ist.

Ziel der vorliegenden Erfindung war es, ein Anreicherungsregime zu finden, das eine möglichst sichere und bevorzugt schnelle Vorkultivierung gewährleistet, um durch die Vermehrung in Quantität und Qualität ausreichend Salmonellen bereitzustellen. Dies trifft insbesondere auf Salmonellen der relevanten Serotypen zu, so dass der eigentliche Spezies-Nachweis ermöglicht wird. Bevorzugt sollte der Spezies-Nachweis auch in Schnellmethoden ermöglicht werden, so dass neben einer möglichen Zeitersparnis im Rahmen der Vorkultivierung weitere Zeitersparnis durch das eigentliche Nachweisverfahren ermöglicht wird.

Außerdem war es Ziel der vorliegenden Erfindung, mit möglichst großer Sicherheit vitale Salmonellen, insbesondere der relevanten Serotypen, in ausreichender Quantität und Qualität für den eigentlichen Spezies-Nachweis zur Verfügung zu stellen, wenn kultivierbare, d.h. im mikrobiologischen Sinne lebende, also auch subletal geschädigte Salmonellen, in der Probe vorhanden sind.

Gelöst wird die Aufgabe der Erfindung durch ein Verfahren zum Erzeugen eines Probensatzes für ein Nachweisverfahren für *Salmonella enterica* ssp. *enterica,* umfassend die Schritte:
a) Gewinnen einer Probe, die potentiell *Salmonella enterica* enthält,
b) Inkubieren der Probe in einem Voranreicherungsmedium für *Salmonella enterica,* so dass eine Anreicherung an Mikroorganismen erfolgt
c) Inkubieren einer Probe des Voranreicherungsmediums in wenigstens einem Anreicherungsmedium für *Salmonella enterica* so dass eine Anreicherung von *Salmonella enterica* erfolgt sofern vorhanden und
d) Gewinnen je einer Probe aus dem Voranreicherungsmedium und dem Anreicherungsmedium oder den Anreicherungsmedien zum Einsatz in einem spezifischen Nachweisverfahren.

Ein Voranreicherungsmedium für *Salmonella enterica* im Sinne der vorliegenden Erfindung ist ein Medium, das im Stand der Technik für die Vorkultur von *Salmonella enterica* und bevorzugt für *Salmonella enterica* ssp. *enterica* vorbeschrieben als Vorkulturmedium ist. Diese Vorkulturmedien sind im Regelfall nicht spezifisch und dienen insbesondere dazu, subletal geschädigte Keime in einen Status zu überführen, indem sie wieder vermehrungsfähig sind und diese dann zu vermehren.

Ein Anreicherungsmedium für *Salmonella enterica* im Sinne der vorliegenden Erfindung ist ein im Stand der Technik beschriebenes Medium für die Anreicherung von *Salmonella enterica,* insbesondere ssp. *enterica,* das nach einem Voranreicherungsschritt für die Anreicherung der betroffenen Keime eingesetzt werden kann. Häufig sind Anreicherungsmedien spezifische Medien, die einen Selektionsdruck auf andere als die nachzuweisenden Keime ausüben. Sie können aber auch sogenannte unspezifische Medien sein, die eine allgemeine Vermehrung von *Salmonella enterica,* insbesondere ssp. *enterica,* und ganz besonders der relevanten Serotypen (Serovare) fördert.

Überraschenderweise hat sich herausgestellt, dass sich die Nachweissicherheit erheblich erhöht, wenn in das nachfolgende spezifische Nachweisverfahren nicht nur eine Probe aus dem zweiten Anreicherungsschritt (Anreicherungsmedium) eingesetzt wird, sondern stattdessen auch noch zusätzlich eine Probe aus der Vorinkubation (Voranreicherungsmedium). Es ließ sich nämlich überraschenderweise eine relevante Anzahl an Proben nachweisen, bei denen nur das Voranreicherungsmedium einen positiven Befund belegt hat.

Dabei ist der Einsatz einer Probe aus der Vorkultur in das spezifische Nachweisverfahren im Regelfall ohne Zeitverlust möglich, da die spezifischen Nachweisverfahren für die einzelnen Proben parallel durchgeführt werden können.

Bevorzugt ist, dass die Erstanreicherung (Schritt b)) für 8 bis 24 Stunden, weiter bevorzugt für 10 bis 22 Stunden, und besonders bevorzugt für 11 bis 20 Stunden, erfolgt.

Ebenfalls bevorzugt ist, dass die Anreicherung in Schritt c) (Zweitanreicherung) für 2 bis 8 Stunden, bevorzugt 3 bis 7 Stunden, und besonders bevorzugt 4 bis 6 Stunden, erfolgt.

Bevorzugt werden für die Erstanreicherung ein Volumen oder ein Gewichtsteil der Probenmatrix zu 9 Anteilen Voranreicherungsmedium eingesetzt und/oder für die Zweitanreicherung (Schritt c) 1 Volumenanteil Voranreicherungsmedium (nach Durchführung der Voranreicherung) mit 19 Volumenanteilen Anreicherungsmedium gemischt.

Bevorzugt ist ein erfindungsgemäßes Verfahren, wobei im Schritt c) wenigstens ein Selektiv-Anreicherungsmedium für *Salmonella enterica* eingesetzt wird.

Besonders bevorzugt ist in diesem Zusammenhang, dass ein Selektiv-Anreicherungsmedium für *Salmonella enterica* ssp. *enterica* eingesetzt wird.

Ein Selektiv-Anreicherungsmedium ist dabei ein solches, das auf andere Keime als die zu selektierenden (hier *Salmonella enterica*) einen Selektionsdruck ausübt, so dass die zu testenden Keime bevorzugt werden. Selektiv-Anreicherungsmedien sind im Stand der Technik bekannt und dementsprechend sind die im Stand der Technik vorbeschriebenen Selektiv-Anreicherungsmedien für *Salmonella enterica,* insbesondere für die bevorzugte Sub-Spezies und ganz besonders für die bevorzugten Serotypen (siehe weiter unten) auch Selektiv-Anreicherungsmedien im Sinne der vorliegenden Erfindung.

Es hat sich herausgestellt, dass, wenn im Schritt c) wenigstens ein Selektiv-Anreicherungsmedium eingesetzt wird, die Ergebnisse deutlich an Zuverlässigkeit gewinnen.

Weiter bevorzugt im Sinne der vorliegenden Erfindung ist es, im Schritt c) des erfindungsgemäßen Verfahrens wenigstens zwei Anreicherungsmedien parallel einzusetzen und so die Zahl der in das spezifische Nachweisverfahren einzusetzenden Proben auf mindestens drei zu erhöhen. Weiter bevorzugt ist in diesem Zusammenhang, im Schritt c) wenigstens ein spezifisches und ein nicht-spezifisches Anreicherungsmedium für *Salmonella enterica* einzusetzen.

Überraschenderweise hat dieses Anreicherungsregime aus einer (unspezifischen) Vorkultur und sowohl einer spezifischen als auch nicht-spezifischen Zweitanreicherung zu besonders zuverlässigen Testergebnissen geführt. Dies ist insbesondere deshalb überraschend, weil zu erwarten wäre, dass für die Zweitanreicherung zwei spezifische (zueinander unterschiedliche) Anreicherungsmedien zuverlässigere Ergebnisse zeigen sollten als solche aus nur einem spezifischen Zweit-Anreicherungsmedium und einem unspezifischen Zweit-Anreicherungsmedium.

Bevorzugt im Sinne der vorliegenden Erfindung ist, dass es sich bei dem Nachweisverfahren, für das die in Schritt d) gewonnenen Proben eingesetzt werden, um einen Nachweis handelt für einen oder mehrere Salmonella-Serotypen ausgewählt aus der Gruppe bestehend aus *Salmonella enteritidis, Salmonella infantis, Salmonella hadar, Salmonella typhimurium* (inkl. der var. Copenhagen und monophasischer ST mit der Antigenformel 1,4,[5],12:-) und *Salmonella virchow.*

Das erfindungsgemäße Verfahren hat sich als besonders geeignet für den Nachweis der vorgenannten Salmonella-Serotypen (Serovare) erwiesen.

Ganz besonders geeignet ist das erfindungsgemäße Verfahren dabei zur Erzeugung für Probenmaterial für ein Nachweisverfahren für *Salmonella enteritidis* und/oder *Salmonella typhimurium,* wobei *Salmonella typhimurium* ganz besonders bevorzugt ist.

Bevorzugt ist ein erfindungsgemäßes Verfahren, wobei das Medium oder die Medien in Schritt c) ausgewählt ist aus der Gruppe bestehend aus Rappaport-Vassiliadis Medium, Rappaport-Vassiliadis Medium mit Soja, Tetrathionat Brilliantgrün Medium, Selenit Bouillon, Selenit Cystein Bouillon; Müller-Kauffmann Tetrathionat-Bouillon mit/ohne Novobiocin als selektives Supplement, Tetrathionat Bouillon, Brain Heart Infusion Bouillon, Hirn-Herz (-Glucose) Bouillon, Selenit-Brilliantgrün Bouillon, Selenit-Brilliantgrün-Sulfapyridin-Bouillon, Lauryl Sulfat Bouillon, MacConkey Bouillon, Mossel Bouillon, modifiziertes halbfestes Rappaport-Vasiliadis Medium, Xylose-Lysin-Desoxycholat-Agar, Rambach-Agar, Caseinsojapepton-Agar, Brilliant-Grün-Agar, CHROMagar Salmonella, Desoxycholat Hydrogen Sulfide Lactose Agar, Desoxycholat Tiple Sugar Iron, Lysine Indol Motility und Bismuth Sulfit Agar und/oder das Medium in Schritt b) ausgewählt ist aus der Gruppe bestehend aus: gepuffertem Peptonwasser (bevorzugt ASU L00.00-20 / ISO 6579), Lactose Bouillon, Trypton Soja Bouillon, Nährbouillon (gemäß den Vorschriften der Food and Drug Administration / FDA der USA), Peptonnährbouillon Universal Voranreicherungs Bouillon und Standard-I- Nährbouillon.

Es hat sich herausgestellt, dass Kombinationen der voraufgeführten Medien zu besonderes zuverlässigen und schnellen Ergebnissen führen.

Nachfolgend werden noch zusätzliche Informationen zu einzelnen Medien gegeben, so z.B. Herstellerangaben und parallel verwendete Bezeichnungen für die entsprechenden Medien:
Voranreicherungsmedien
   - gepuffertes Peptonwasser (Oxoid, Sigma-Aldrich) nicht-selektiv (animal or vegetable derived peptone) = Tryptone Phosphate Water
   - Tryptose-Soja-Bouillon (TSB) = CASO nicht-selektiv
   - Universal Voranreicherungsbuillon, Universal Pre-enrichment Broth (UPB) (Difco, Sigma-Alrdich) nicht-selektiv
   - Revive preenrichement-medium for Salmonella (Ampcor Diagnostics) nicht-selektiv 2 hh, danach in selektives SC
   - Salmonella Enrichment Broth I (Foss Electric, DK) selektiv
   - ONE Broth - Salmonella (OXOID)
Zweit-Anreicherungsmedien
   a) Flüssigmedien
      - Rappaport-Vassiliadis Medium (RV) (Oxoid, Sigma-Aldrich) = Magnesiumoxid-Malachitgrün-Bouillon, Modifizierte Rappaport Vasilliadis Bouillon, Salmonella enrichment broth acc. to Rappaport & Vasilliadis, selektiv
      - Rappaport-Vassiliadis Medium mit Soja (RVS) (Sigma-Aldrich), Nährstoff-reicher (acc. to DIN EN ISO 6579:2002), selektiv
      - Tetrathionate Brilliantgrün Medium (TT), Tetrathionate Brilliant Green Bile Enrichment Broth (TBG) (Difco, Sigma-Aldrich), selektiv
      - Selenite Broth, Selenite Cysteine broth (SC) (Oxoid, Merck, Sigma-Aldrich); Selenit-F Broth (BD), Mannitol Selenite Broth (Oxoid), selektiv
      - Müller-Kauffmann Tetrathionat(-Novobiocin)-Bouillon (MKTT(n)) (Oxoid, mit/ ohne Novobiocin als selektives Supplement, selektiv
      - Tetrathionate Broth (Oxoid), selektiv
      - Brain Heart Infusion Broth (BHI), Hirn-Herz-Bouillon (Oxoid), nicht-selektiv
      - Lauryl Sulfate Broth = Lauryl Casein peptone Broth
      - MacConkey Broth (coliform)
      - Mossel broth = Buffered Glucose-Brilliant Green Bile Broth acc. to Mossel
Halbfestes Medium
   - Modified Semisolid Rappaport-Vasiliadis Medium MSRV
Festmedien
   - Xylose-Lysin-Desoxycholat-Agar (XLD)
   - Xylose-Lysin-Brillantgrün-Agar (XLBG)
   - Caseinsojapepton-Agar (CASO-/ TSA-Agar) =TSI?
   - Brilliant-Grün-Agar (BGA) (Oxoid) = Brillantgrün-Phenolrot-Laktose-Saccharoses-Agar (BPLS)(selektiv)
   - Mannit-Lysin-Kristallviolett-Brillantgrün-Agar (MLCB)
   - Lysin-Mannitol-Glycerol-Agar (LMG)
   - Xylose-Lysin-Tergitol4-Medium (XLT4)
   - Hektoen-Enteric-Agar (HE)
   - Novobiocin-Brillantgrün-Lactose-Medium
   - CHROMagar Salmonella
   - Rambach-Agar (Merck)Desoxycholat Hydrogen Sulfid Lactose Agar (DHL)
   - Desoxycholat
   - Tiple Sugar Iron (TSI)
   - Lysine Indol Motility (LIM)
   - Bismuth Sulfit Agar (selektiv)

Diese zusätzlichen Angaben können dem Fachmann helfen, die geeigneten Medien auszuwählen.

Bevorzugt ist ein erfindungsgemäßes Verfahren, wobei das Medium in Schritt b) gepuffertes Peptonwasser und/oder wenigstens ein Medium in Schritt c) Brain Heart Infusion Bouillon oder Rappaport-Vassiliadis Medium mit Soja ist.

Ganz besonders bevorzugt in diesem Zusammenhang ist, im Schritt c) Brain Heart Infusion Bouillon als nicht-selektives Medium und Rappaport-Vassiliadis-Medium mit Soja als selektives Medium einzusetzen.

Es hat sich nämlich herausgestellt, dass mit einer Vorkultur in gepuffertem Peptonwasser und Zweitkulturen in sowohl Brain Heart Infusion Bouillon als auch in Rappaport-Vassiliadis-Medium mit Soja Proben gewonnen werden, die für das nachfolgende spezifische Nachweisverfahren die zuverlässigste Grundlage bieten. Hierbei war es sogar möglich, ein falsch negatives Ergebnis des anschließenden spezifischen Nachweises, insbesondere für die weiter unten bevorzugt beschriebenen spezifischen Nachweise zu 100% auszuschließen. Dies gilt insbesondere für den Salmonella-Serovar *S. thyphimurium* (ST).

Mit anderen Worten, wenn kultivierbare ST in den Proben waren, wurden sie auch erfolgreich mit dem ganz besonders bevorzugten Anreicherungsregime in dem anschließenden spezifischen Nachweis detektiert.

Teil der Erfindung ist auch ein Verfahren zum Nachweis von Salmonella enterica ssp. enterica, umfassend die Schritte i) Durchführen eines erfindungsgemäßen Verfahrens zum Erzeugen eines Probensatzes und ii) Einsetzen aller Proben aus Schritt d) des Verfahrens in ein Nachweisverfahren für *Salmonella enterica* ssp. *enterica,* wobei das Ergebnis des Nachweises positiv ist, sofern mindestens eine der Proben im Nachweisverfahren ein positives Ergebnis ergibt.

Insbesondere wenn die bevorzugten erfindungsgemäßen Verfahren zum Erzeugen eines Probensatzes angewendet werden, lassen sich in dem erfindungsgemäßen Verfahren zum Nachweis von Salmonellen schnell äußerst zuverlässige Testergebnisse erzielen.

Bevorzugt ist ein erfindungsgemäßes Verfahren zum Nachweis für Salmonella enterica ssp. enterica, wobei das in Schritt ii) durchzuführende Nachweisverfahren ausgebildet aus der Gruppe bestehend aus kulturellem Nachweis (z.B. nach EU VO 6579, Spezies-spezifische Nährmedien), Nukleinsäure-Detektionsverfahren (nach Amplifizierung), insbesondere (real-time) PCR, LAMP, Microarray, Sequenzierung sowie DNA-Hybridisierung, Antigen-Antikörper-Reaktionen, insbesondere ELISA und verwandte Assays, RIM/ lateral flow strip, Fluoreszmikroskopie (Visual Immunoassay/ VIA), Immunomagnetische Separation sowie Agglutination mit Antiseren und phagenbasiertem Nachweis.

Besonders bevorzugte Nachweisverfahren, für die der im erfindungsgemäßen Verfahren erzeugte Probensatz besonders geeignet ist, sind dabei Nukleinsäure-Detektionsverfahren (ggf. nach Amplifizierung) und besonders bevorzugt PCR-Verfahren, wobei real-time PCR ganz besonders bevorzugt ist.

Bei diesen bevorzugten und ganz besonders bevorzugten Verfahren wirkt sich der Vorteil der Zuverlässigkeit des im erfindungsgemäßen Verfahren erzeugten Probensatzes ganz besonders aus. Zudem sind insbesondere die Nukleinsäure-Detektionsverfahren schnell durchzuführen, so dass durch Kombination des erfindungsgemäßen Kulturregimes mit einem schnellen spezifischen Nachweis ein überdurchschnittlich zuverlässiges Ergebnis überdurchschnittlich schnell erhalten werden kann.

Teil der Erfindung ist auch die Verwendung eines Satzes von Proben aus Schritt d) des erfindungsgemäßen Verfahrens zum Erzeugen eines Probensatzes in einem Nachweis für *Salmonella enterica* ssp. *enterica,* wobei besonders bevorzugt sich das Nachweisverfahren auf ein oder mehrere Salmonella-Serotypen, ausgewählt aus der Gruppe bestehend aus *Salmonella enteritidis, Salmonella infantis, Salmonella hadar, Salmonella typhimurium* und *Salmonella virchow* und ganz besonders bevorzugt auf einen Nachweis von *Salmonella enteritidis* und/oder *Salmonella typhimurium* und am meisten bevorzugt auf *Salmonella typhimurium* bezieht.

Teil der Erfindung ist auch ein Kit umfassend ein Medium für Schritt b) und wenigstens ein Medium für Schritt c) jeweils wie oben definiert.

Weiter bevorzugt umfasst das erfindungsgemäße Kit zwei oder drei der weiter oben als bevorzugt beschriebenen Medien (wobei stets ein Medium ein Medium für Schritt b) ist).

Besonders bevorzugt umfasst das erfindungsgemäße Kit gepuffertes Peptonwasser, Rappaport-Vassiliadis-Medium mit Soja und Brain Heart Infusion Bouillon bzw. die Grundlagen für diese Medien.

Die Medien in den erfindungsgemäßen Kits sind, wie sich aus dem Vorbeschriebenen ergibt, besonders geeignet für die Durchführung der erfindungsgemäßen Verfahren.

### Beispiele

### Beispiel 1

Erzeugen eines Probensatzes für ein Nachweisverfahren für *Salmonella enterica* ssp. *enterica*

Die Gewinnung des zu untersuchenden Materials erfolgte nach üblichen Methoden, z.B. nach Hühner-Salmonellen-VO aus 2011, VO (EG) 2160 aus 2003 oder VO (EG) 178 aus 2002. Die zu testende Probe wurde in einer neunfachen Menge mit gepuffertem Peptonwasser (ASU L00.00-20/ISO6579, Hersteller Sigma-Aldrich oder gleichwertig) in einem Stomacherbeutel aufgenommen und homogenisiert. Dabei wurde das eingesetzte gepufferte Peptonwasser (und auch die weiteren später eingesetzten Medien) auf die finale Inkubationstemperatur vor Start der Inkubationsperiode vorgewärmt.

Es folgt eine Inkubation bei 37±1°C ohne Schütteln für 18±2 Stunden. Für Proben aus frischem Schweinefleisch und Geflügelfleisch konnte die Dauer der Vorkultur in gepuffertem Peptonwasser auf 15±2 Stunden gesenkt werden, für Nackenhautproben von Geflügel sogar auf 13±2 Stunden. Dabei führte die Senkung der Vorinkubationszeit zu gleichwertigen Ergebnissen im nachfolgenden Nachweisverfahren.

Als zweiter Anreicherungsschritt erfolgte ein Transfer von je 0,5 ml aus dem Voranreicherungsansatz nach Inkubation in jeweils 10 ml Brain Heart Infusion (Firma Oxoid) und Rappaport-Vassiliadis-Medium mit Soja (Sigma Aldrich). Bei stark gewebehaltigen oder fetthaltigen Proben wurden Stomacherbeutelmit Filter eingesetzt.

Die zweiten Anreicherungskulturen wurden ohne Schütteln für wenigstens 5 Stunden bei 37±1°C (Brain Heart Infusion Medium) und 43±1°C Rappaport-Vassiliadis-Medium mit Soja durchgeführt.

Nach dieser Inkubationszeit wurden aus der Vorkultur und den beiden zweiten Anreicherungsschritten jeweils Proben von 1 ml in ein 1,5 ml-Probengefäß überführt. Diese drei Proben bildeten den Probensatz für das nachfolgende spezifische Nachweisverfahren.

### Beispiel 2

### Durchführung des spezifischen Nachweisverfahrens

Der Probensatz aus Beispiel 1 wurde nachfolgend mit einer modifizierten Salmonella ssp.-spezifischen real-time PCR in Anlehnung an ASU L00.00-98 gemäß §64 LFGB und parallel oder nachfolgend mit einer ST-spezifischen real-time PCR einem spezifischen Nachweis auf Salmonella ST unterworfen. Dazu wurde eine DNA-Extraktion durchgeführt und anschließend die PCR-Nachweise. Dabei zeigte sich, dass die besten und zuverlässigsten Testergebnisse erzielt werden, wenn alle drei Proben des Probensatzes eingesetzt wurden.

Allgemein zeigte sich, dass in etwas über 90% der positiven Proben in allen drei Anreicherungsmedien Salmonella typhimurium (ST) nachweisbar war, aber in den übrigen Fällen in wenigstens 1% der einzelnen der drei Einzelproben ein Nachweis nicht erfolgreich war, obwohl die ursprüngliche Probe positiv war. Zum Teil war der einzige positive Nachweis in der Probe der Vorkultur (aus gepuffertem Peptonwasser) zu finden, wobei auch in einem solchen Falle der Nachweis als positiv gewertet wurde.

Diese Wertung zeigte sich als gerechtfertigt, gesichert durch Nachweis auf Festmedien.

## Patentansprüche

1. Verfahren zum Erzeugen eines Probensatzes für ein Nachweisverfahren für *Salmonella enterica* ssp. *enterica,* umfassend die Schritte:
a) Gewinnen einer Probe, die potentiell *Salmonella enterica* enthält,
b) Inkubieren der Probe in einem Voranreicherungsmedium für *Salmonella enterica,* so dass eine Anreicherung an Mikroorganismen erfolgt,
c) Inkubieren einer Probe des Voranreicherungsmediums in wenigstens einem Anreicherungsmedium für *Salmonella enterica,* so dass eine Anreicherung von *Salmonella enterica* erfolgt sofern vorhanden und
d) Gewinnen je einer Probe aus dem Voranreicherungsmedium und dem Anreicherungsmedium oder den Anreicherungsmedien zum Einsatz in einem spezifischen Nachweisverfahren.

2. Verfahren nach Anspruch 1, wobei in Schritt c) wenigstens ein Selektivanreicherungsmedium für *Salmonella enterica* eingesetzt wird.

3. Verfahren nach Anspruch 1 oder 2, wobei es sich bei dem Nachweisverfahren um einen Nachweis handelt für einen oder mehrere Salmonella-Serotypen, ausgewählt aus der Gruppe bestehend aus *Salmonella enteritidis, Salmonella infantis, Salmonella hadar, Salmonella typhimurium* und *Salmonella virchow.*

4. Verfahren nach Anspruch 3, wobei es sich bei dem Nachweisverfahren um einen Nachweis handelt für *Salmonella enteritidis* und/oder *Salmonella typhimurium.*

5. Verfahren nach einem der vorangehenden Ansprüche, wobei in Schritt c) wenigstens ein Selektivanreicherungsmedium für *Salmonella enterica* und wenigstens ein nicht-selektives Medium eingesetzt wird.

6. Verfahren nach einem der vorangehenden Ansprüche, wobei das Medium oder die Medien in Schritt c) ausgewählt ist aus der Gruppe bestehend aus Rappaport-Vassiliadis Medium, Rappaport-Vassiliadis Medium mit Soja, Tetrathionat Brilliantgrün Medium, Selenit Bouillon, Selenit Cystein Bouillon; Müller-Kauffmann Tetrathionat-Bouillon mit/ohne Novobiocin als selektives Supplement, Tetrathionat Bouillon, Brain Heart Infusion Bouillon, Hirn-Herz (-Glucose) Bouillon, Selenit-Brilliantgrün Bouillon, Selenit-Brilliantgrün-Sulfapyridin-Bouillon, Lauryl Sulfat Bouillon, MacConkey Bouillon, Mossel Bouillon, modifiziertes halbfestes Rappaport-Vasiliadis Medium, Xylose-Lysin-Desoxycholat-Agar, Rambach-Agar, Caseinsojapepton-Agar, Brilliant-Grün-Agar, CHROMagar Salmonella, Desoxycholat Hydrogen Sulfide Lactose Agar, Desoxycholat Tiple Sugar Iron, Lysine Indol Motility und Bismuth Sulfit Agar
und/oder das Medium in Schritt b) ausgewählt ist aus der Gruppe bestehend aus:
gepuffertem Peptonwasser, Lactose Bouillon, Trypton Soja Bouillon, Nährbouillon, Peptonnährbouillon Universal Voranreicherungs Bouillon und Standard-I-Nährbouillon.

7. Verfahren nach einem der vorangehenden Ansprüche, wobei das Medium in Schritt b) gepuffertes Peptonwasser und/oder wenigstens ein Medium in Schritt c) Brain Heart Infusion Bouillon oder Rappaport-Vassiliadis Medium mit Soja ist.

8. Verfahren zum Nachweis für *Salmonella enterica* ssp. *enterica,* umfassend die Schritte:
i) Durchführen eines Verfahrens nach einem der Ansprüche 1 bis 7 und
ii) Einsetzen aller Proben aus Schritt d) des Verfahrens nach einem der Ansprüche 1 bis 7 in ein Nachweisverfahren für *Salmonella enterica* ssp. *enterica,* wobei das Ergebnis des Nachweises positiv ist, sofern mindestens eine der Proben im Nachweisverfahren ein positives Ergebnis ergibt.

9. Verfahren nach Anspruch 8, wobei das in Schritt ii) durchzuführende Nachweisverfahren ausgewählt ist aus der Gruppe bestehend aus:
kulturellem Nachweis (z.B. EU VO 6579, Spezies-spezifische Nährmedien),
Nukleinsäure-Detektionsverfahren (nach Amplifizierung), insbesondere (real-time) PCR, LAMP, Microarray, Sequenzierung sowie DNA-Hybridisierung, Antigen-Antikörper-Reaktionen, insbesondere ELISA und verwandte Assays, RIM/ lateral flow strip, Fluoreszmikroskopie (Visual Immunoassay/ VIA), Immunomagnetische Separation sowie Agglutination mit Antiseren und phagenbasiertem Nachweis.

10. Verwendung eines Satzes Proben aus Schritt d) eines der Verfahren nach einem der Ansprüche 1 bis 7 in einem Nachweis für *Salmonella enterica* ssp. *enterica.*

11. Kit für ein Verfahren nach einem der Ansprüche 1 bis 9, umfassend ein Medium für Schritt b) und wenigstens ein Medium für Schritt c) wie jeweils in Anspruch 6 definiert.
